Europäisches Patentamt

(19) European Patent Office

Office européen des brevets

(11) Publication number: **0 152 390**
**A2**

(12) # EUROPEAN PATENT APPLICATION

(21) Application number: **85850050.7**

(22) Date of filing: **12.02.85**

(51) Int. Cl.⁴: **A 61 C 17/04**
**A 61 M 1/00, A 47 L 9/02**

(30) Priority: **13.02.84 SE 8400747**

(43) Date of publication of application:
**21.08.85 Bulletin 85/34**

(84) Designated Contracting States:
**AT BE CH DE FR GB IT LI NL SE**

(71) Applicant: **Mediplast AB**
**Rasundavägen 60**
**S-171 52 Solna(SE)**

(72) Inventor: **Andersson, Ingvar**
**Pl. 80**
**S-450 34 Fiskebäckskil(SE)**

(74) Representative: **Roth, Ernst Adolf Michael et al,**
**GÖTEBORGS PATENTBYRA AB Box 5005**
**S-402 21 Göteborg(SE)**

(54) **Suction device.**

(57) A suction nozzle which when being stopped up automatically delimits the suction force in the suction opening (2) to a certain predetermined value independently of the negative pressure in the suction conduit (1, 12). The suction nozzle comprises a sleeve member (3) arranged at the front end of the suction conduit (1, 12) and which forms the suction opening (2) of the device. The sleeve member (3) is by a resilient member (4) held in a first position, suction position, in which it covers side passages (5,6;15) to the suction conduit (1) and in which suction is performed through the suction opening (2). If the pressure difference immediately inside and outside the suction opening (2) exceeds a certain value, e.g. if the suction opening is stopped up, the sleeve member (3) is against the action of the resilient member (4) automatically displaced to a second position in which said side passages (5,6;15) to the suction conduit are opened, thereby reducing the suction force in the suction opening (2).

./...

# FIG 8

# SUCTION DEVICE

## Background of the invention

The present invention refers to a suction device comprising a suction conduit and a suction source connected thereto and further comprising a sleeve member arranged at the front end of the suction conduit and forming the suction opening of the device, said sleeve member is limitedly displaceable in axial direction from a first position, in which it covers side passages to the suction conduit, to a second position in which said side passages are exposed.

Suction devices of various kinds connected to a vacuum pump are used in for example surgery and dental care. One problem arising in this connection is that the nozzle opening may touch body tissues so that these will adhere to the nozzle by suction. Since upon such an occasion the vacuum pump continues to evacuate air out of the suction conduit, the pressure will be further reduced and the nozzle will adhere even more firmly. The result will be, on one hand, that tissues may be injured and, on the other hand, that the nozzle opening will be stopped up by loose objects.

This problem can be solved in different ways. One way is to provide the suction conduit with a valve, or another member, which when the pressure difference between the atmosphere and the suction conduit exceeds a certain value, will open a flow connection between the suction conduit and the atmosphere. In this way the pressure in the suction conduit is never allowed to reach below this value, so that the risk of the nozzle adhering by suction will be considerably reduced. One drawback is however, that the valve also opens when said pressure difference gets below this value due to the fact that large liquid amounts are sucked up through the suction conduit.

The problem of stopping up of the suction nozzle also occurs in many other connections, e.g. in vacuum cleaners which can adhere to rugs and carpets and the like.

In US-A-3,913,577 is disclosed an aspirator for dental and surgical applications and having a tip comprising a tubular inner core with radial openings and a sleeve member telescoped over the core. The sleeve member also has radial openings and by displacing and rotating the sleeve with respect to the core, the openings are exposed or closed in order to provide a manual selection between greater suction or less suction in the aspirator tip. No automatic reduction of the suction force in the aspirator tip is however provided if the tip would adhere by suction to the tissues.

## Summary of the invention

The object of the present invention is to provide a suction nozzle which, when being stopped up, e.g. adhered by suction, automatically reduces the suction force in the suction opening to a certain predetermined value independently of the negative pressure in the suction conduit. This has according to the invention been achieved by the fact that a resilient member is arranged to hold the sleeve member in said first position, in which suction is performed through the suction opening and that the sleeve member against the action of the resilient member due to a pressure difference immediately inside and outside the suction opening is arranged to automatically be displaced to said second position, in which the side passages to the suction conduit are opened, at which the suction force in the suction opening is reduced.

## Description of the drawings

The invention will now be closer described with reference to some embodiments shown in the accompanying drawings.

Figures 1 and 2 show sections through a first embodiment of the suction nozzle according to the invention in suction position and in a position where the suction opening has been stopped up.

Figures  3  and 4 show sections through a second embodiment in suction position and stopped up position respectively.

Figures 5 and 6 show sections through a  third  embodiment  in suction position and stopped up position respectively.

Figures 7  and  8 show sections through a fourth embodiment in suction position and stopped up position respectively.

Figure 9 is a section according to the line IX-IX in figure 7.

Figure 10 shows in detail a groove provided in  a  portion  of the suction conduit.


Description of the embodiments


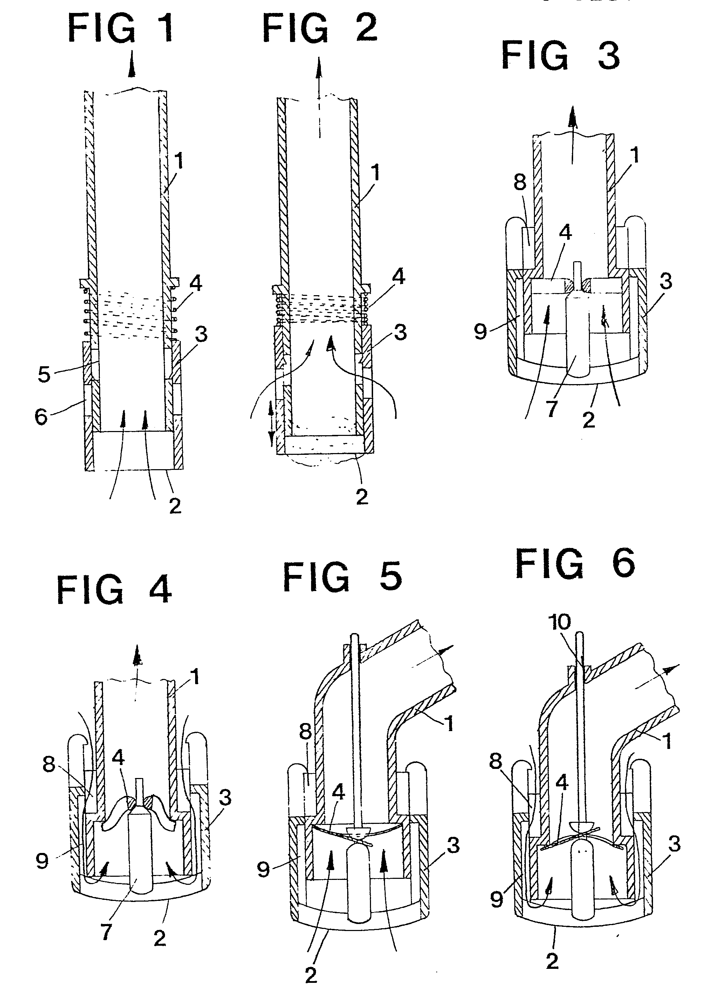

The  suction  conduit  is  denoted  with  the numeral 1 and is connected to a suction source not shown. At the front  end  of the  suction  conduit on the outside thereof a sleeve member 3 is arranged, which at its front end, the  suction  opening  2, possibly  can  be  provided  with  a  lattice or the like. The sleeve member 3 according to the embodiment shown in figure  1 and  2 is by a spring 4 held in the position shown in figure 1 and can  against  the  action  of  said  spring  4  be  pushed backwards  to the position shown in figure 2. The suction con duit 1 as well as the sleeve member 3 is provided with  radial passages  5  and 6 respectively, which in the suction position shown in figure 1 are closed, at which the device  only  sucks through  the  suction  opening  2.  If,  however,  the suction opening 2 would be stopped up of a loose object or  adhere  by suction  the  sleeve  member  3  will,  due  to  the  pressure difference inside and outside the suction opening 2, be pushed backwards  against  the action of the spring 4 to the position shown in figure 2, in which the radial passages 5 and  6  will be located just opposite each other and open a side passage to the suction conduit. The suction force in the suction  opening 2 is by that reduced or completely ceased, so that the adhered object is released. The device can, however, continue to  suck through the side passages 5 and 6.


It is important to notice that the  sleeve  member  3  is  not actuated  by the negative pressure in the vacuum system, which also is dependent on the flow speed in the suction conduit and thus increases upon suction of large liquid amounts, for example. The sleeve member 3 is actuated firstly when the pressure difference immediately outside and inside the suction opening due to a stop up rises above a certain value corresponding to the spring force of the resilient element 4.

According to the embodiment shown in figure 3 and 4 the resilient member 4 consists of a disk of rubber or another resilient material, which is attached about a pin 7 fixedly connected to the sleeve member 3 at the lattice provided at the suction opening thereof. In the suction position shown in figure 3 the suction conduit communicates with the environment only through the suction opening 2, while in the position where it has been stopped up (fig. 4) and the sleeve member 3 has been pushed backwards against the action of the resilient disk 4, side passages 8 and 9 between the suction conduit 1 and the sleeve member 3 are exposed and admit communication between the suction conduit 1 and the environment.

The embodiment shown in figure 5 and 6 differs from the one according to figure 3 and 4 by the fact that the resilient member 4 consists of a so-called bistable spring, which in the suction position of the device (fig. 5) takes one of its stable positions and in the position in which the suction opening has been stopped up and the sleeve member 3 pushed backwards takes its opposite stable position (fig. 6). The return to suction position is made manually by means of a pin 10 extending out through an opening in the suction conduit 1. In both previous embodiments the return to suction position takes place automatically when the pressure action upon the sleeve member 3 is below the spring force.

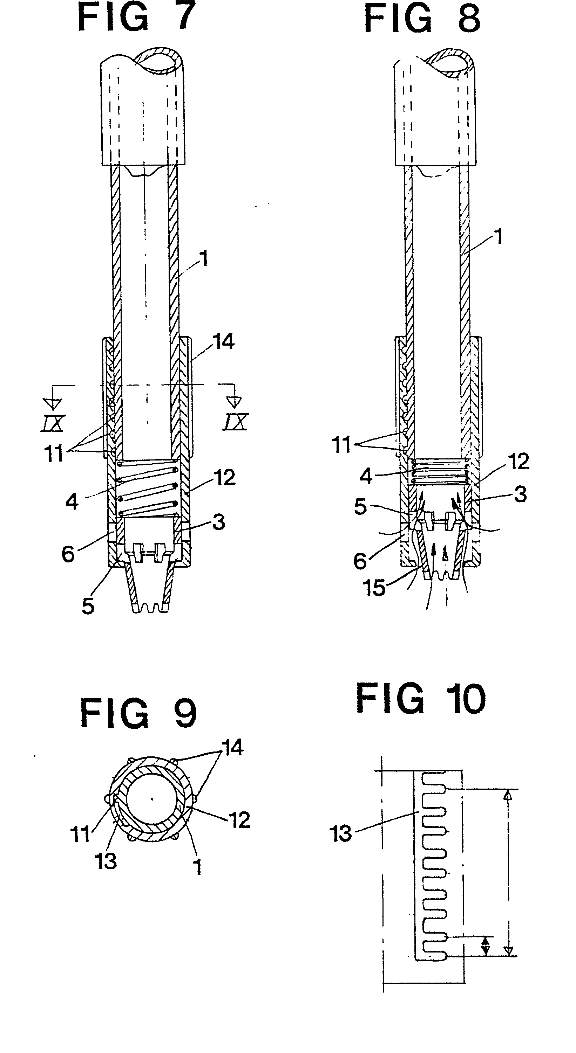

In order to prevent unnecessary opening of the sleeve member and in order to keep a high flow speed through the suction opening the edge surface thereof can be wavy or toothed so that air is permitted to pass, as is shown in fig. 7 and 8. In this embodiment is further permitted a manual adjustment of the prestressing of the spring 4 and by that the opening pressure of the sleeve member 3. When sucking in sensitive

parts of the body, e.g. the brain, it is desired that the opening pressure is relatively low while in other cases considerably higher opening pressures can be tolerated.

The suction conduit 1 is in the shown embodiment provided with a number of external bosses 11 on a certain axial distance from each other, while a tubular piece 12 arranged outside the suction conduit 1 and forming an extension thereof is provided with an internal groove 13 having the design shown in fig. 10. The groove 13 is designed to cooperate with the bosses 11, so that the tubular piece 12 can be locked in a number of different positions with respect to the suction conduit 1. Of course the tubular piece 12 can on its inside be provided with bosses 11, which can be locked in a groove 13 on the outside of the suction conduit 1. When displacing the tubular piece 12 with respect to the suction conduit 1 the prestressing of the spring 4 is changed. The tubular piece 12 is further provided with a number of external cams 14 forming a grip when manipulating the tubular piece. The prestressing of the spring 4 can of course be adjustable in many other ways than is shown here.

The sleeve member 3 is tapered at its front end, i.e. the suction end, at which openings 15 are formed between the suction conduit 1, in this case the front edge of the tubular piece 12, and the sleeve member 3 in stopped up position when the sleeve member has been pressed backwards according to figure 8. The nozzle can then suck beside the stopped up tip. The radial passages 5 and 6 can possibly in this case be eliminated.

The invention is of course not limited to the embodiments shown but can be varied within the scope of the claims. The device is not limited to suction devices for use in medical care but can be applied at e.g. vacuum cleaners and in all connections with suction devices where stopping up of the suction nozzle is a problem.

CLAIMS

1. A suction device comprising a suction conduit and a suction source connected thereto and further comprising a sleeve member (3) arranged at the front end of the suction conduit (1) and forming the suction opening (2) of the device, said sleeve member is limitedly displaceable in axial direction from a first position, in which it covers side passages (5,6;8,9;15) to the suction conduit (1), to a second position in which said side passages are exposed,
c h a r a c t e r i z e d   i n,
a resilient member (4) arranged to hold the sleeve member (3) in said first position in which suction is performed through the suction opening (2) and that the sleeve member against the action of the resilient member (4) due to a certain pressure difference immediately outside and inside the suction opening (2) is arranged to automatically be displaced to said second position, in which the side passages (5,6;8,9;15) to the suction conduit are opened, at which the suction force in the suction opening (2) is reduced.

2. A suction device as claimed in claim 1,
c h a r a c t e r i z e d   i n,
that the suction opening (2) of the sleeve member (3) is toothed or wavy.

3. A suction device as claimed in claim 1 or 2,
c h a r a c t e r i z e d   i n,
that the sleeve member (3) shows a portion having a reduced cross-section at the suction opening (2), so that in said second position side passages (15) are formed between the front edge of the suction conduit (1;12) and the sleeve member.

4. A suction device according to any of the preceding claims,
c h a r a c t e r i z e d   i n,
that the prestressing of the resilient element (4) is adjustable.

0152390

FIG 1 FIG 2 FIG 3

FIG 4 FIG 5 FIG 6

0152390

# FIG 7

# FIG 8

# FIG 9

# FIG 10